# EUROPEAN PATENT APPLICATION

(11) **EP 4 647 073 A1**
(43) Date of publication of application: **12.11.2025**
(21) Application number: 25174279.7
(22) Date of filing: 05.05.2025
(51) Int. Cl.: A61K 31/407, A61K 31/403, A61K 31/015, A61P 11/00, A61K 36/75, A61K 9/68, A61K 9/00

(54) **MURRAYA KOENIGII IN THE TREATMENT OF UPPER RESPIRATORY TRACT INFECTIONS**

(30) Priority: 08.05.2024 IT 202400010384
(71) Applicant: Nutraceutical Technology Pharma Biotech S.r.l., 64020 Castellalto (TE) (IT)
(72) Inventor: Marini, Luca, 64020 Castellalto (TE) (IT)
(74) Representative: Villa, Livia

(57) **Abstract**

Disclosed are carbazole alkaloids, in particular those present in *M. koenigii,* as active agents in the treatment of upper respiratory tract infections ascribable to *S. pyogenes*. A pharmaceutical composition comprising said carbazole alkaloids is also disclosed, the pharmaceutical composition preferably being in the form of a chewing-gum, a chewable tablet, a chewable soft jelly, an oral spray, or a syrup. These products including said plant-derived antimicrobials are found to be desirable alternatives/adjuvants to antibiotics for treating infections, while minimizing antimicrobial resistance.

## Description

### FIELD OF THE INVENTION

The present invention concerns carbazole alkaloids, in particular those present in *Murraya koenigii* (*M. koenigii*), as active agents in the treatment of upper respiratory tract infections ascribable to *Streptococcus pyogenes.* The present invention also concerns a pharmaceutical composition comprising said carbazole alkaloids, the composition preferably being in the form of a chewing-gum, a chewable tablet, a chewable soft jelly, an oral spray, or a syrup. These products including said plant-derived antimicrobials are found to be desirable alternatives/adjuvants to antibiotics for treating infections, while minimizing antimicrobial resistance.

### BACKGROUND ART

Upper respiratory tract infections (URTIs) include nasopharyngitis (common cold), sinusitis, pharyngitis, tonsillitis, laryngitis, tracheitis, obstructive laryngitis (croup), epiglottitis, and laryngopharyngitis. URTIs are one of the most common reasons for seeking medical care globally.

Children are particularly susceptible to URTIs due to their lower immune system memory and their frequent close contact with each other. These infections are of bacterial or viral origin. It is therefore important to identify when antibiotic use is warranted as overuse of antibiotics contributes to Antimicrobial Resistance (AMR). Nevertheless, URTIs are often associated with unjustified antibiotic prescription, especially in children.

AMR threatens the effective prevention and treatment of an ever-increasing range of infections and it has been prioritized by the world health organization (WHO) as one of the top 10 global public health threats facing humanity.

The most common bacterial pathogens responsible for URTIs are group A beta-haemolytic streptococcus (GABHS, *Streptococcus pyogenes*) and *Streptococcus pneumoniae.*

*S. pyogenes* is a gram-positive bacterium and it is considered a pathogen of public health significance. It can cause a wide variety of acute infections, such as soft tissue infections and pharyngitis; severe life-threatening infections, such as streptococcal toxic shock syndrome; and devastating postinfectious sequelae, such as rheumatic fever and glomerulonephritis. The global burden of severe *S. pyogenes* infections is 18.1 million cases, with 1.78 million new cases per year. There are at least 517'000 deaths each year due to severe GABHS diseases. Children, the immunocompromised and the elderly are at the greatest risk of *S. pyogenes* infections.

β-lactam penicillin is the first-line treatment for GABHS infections. This treatment is cost-effective and has a narrow spectrum of activity. In penicillin allergic patients, macrolides are the most commonly used antibiotics. However, the resistance rate to macrolides is gradually increasing, due to the overuse of such antibiotics.

In this scenario, plant-derived antimicrobials could be desirable alternatives/adjuvants to antibiotics for treating infections, while minimizing AMR.

The object of the present invention is therefore to provide plant-based antimicrobials which are effective towards upper respiratory tract infections and associated symptoms, in particular those ascribable to *S. pyogenes.*

### SUMMARY OF THE INVENTION

Said object has been achieved by carbazole alkaloids for use as active agents in the treatment of upper respiratory tract infections ascribable to *Streptococcus pyogenes.*

In another aspect, the present invention concerns a pharmaceutical composition comprising said carbazole alkaloids.

### BRIEF DESCRIPTION OF THE FIGURES

The characteristics and advantages of the present invention will be evident from the following detailed description, the embodiments provided for illustrative and nonlimiting purposes, and the annexed figures wherein:
Fig. 1: Antibacterial activity of six DMSO extracts of *M. koenigii* leaves from different geographical origins against *S. pyogenes.* The well in the centre was filled with DMSO as a negative control.
Fig. 2: Antibacterial activity of DMSO extract of *M. koenigii* leaves against *S. pyogenes.* Three different concentrations were tested: 100 mg/ml (1), 20 mg/ml (2), 5 mg/ml (3), DMSO (CTRL, negative control).
Fig. 3: Picture of streaked M17 agar plates after growing overnight. (A) Wells D1-D4 (extract diluted from 1:100 to 1: 800). (B) Wells D5-D8 (extract diluted from 1:1600 to 1: 12800). (C) Wells H1-H4 (DMSO diluted from 1:2 to 1: 16).
Fig. 4: Picture of the microplate used for the TTC assay. Rows A-C (extract diluted from 50 mg/ml to 0.2 mg/ml). Rows D-F (extract diluted from 10 mg/ml to 0.04 mg/ml). Rows G-H (solvent DMSO diluted from 1:2 to 1: 512). Controls: column 10 (last dilution of each tested extract), column 11 (*S. pyogenes* without extract), column 12 (medium M17 without extract).
Fig. 5: A histogram showing the results of the TTC assay (see also Tab. 2 in Example 3).
Fig. 6: Picture of the microplate used for the TTC assay. Rows A-B (extract diluted from 10 mg/ml to 0.04 mg/ml). Rows G-H (solvent DMSO diluted from 1:2 to 1: 512). Controls: column 10 (last dilution of each tested extract), column 11 (*S. pyogenes* without extract), column 12 (medium M17 without extract).
Fig. 7: A histogram showing the antibacterial activity of amoxicillin alone, extract alone and mix (amoxicillin and extract) against *S. pyogenes* (see also Tab. 3 in Example 4).
Fig. 8: Chromatogram of the Mix Standard Solution, according to the Example 12.
Fig. 9: Percentage of Koenigicine, Koenimbine, Mahanimbine vs. different chewing time points. The single points are the average of the values obtained from the three chewers, according to the Example 12.
Fig. 10: Percentage of total alkaloids release vs. different chewing time points. The single points are the average of the values obtained from the three chewers, according to the Example 12.
Fig. 11: Picture of the M17 agar plate displaying the antibacterial activity of the Koenimbine (1), Koenigicine (2) and Mahanimbine (4) standards against *S. pyogenes* ATCC 19615, according to the Example 13. The well in the centre was filled with DMSO as a negative control.

### DETAILED DESCRIPTION OF THE INVENTION

The present invention concerns therefore carbazole alkaloids for use as active agents in the treatment of upper respiratory tract infections ascribable to *S. pyogenes.*

Carbazole alkaloids are natural compounds of the indole alkaloid type, derived from carbazole:

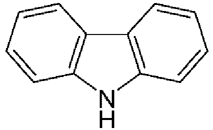

For the purposes of the present invention, with the term "carbazole alkaloids", it is meant to comprise at least one of the following compounds: Olivacine, Ellipticin, Glybomine B, Mahanine, Mahanimbine, Murrayanol, Koenimbine, O-Methylmurrayamine A, Koenigicine, Koenigine, Koenoline, Koenine, Murrayone, Mahanimbicine, Bicyclomahanimbicine, Phebalosin, Isomahanimbine, Koenimbidine, Euchrestine B, Bismurrayafoline E, Isomahanine, Mahanimbinine, Girinimbilol, Pyrayafoline-D, Glycozoline, Cyclomahanimbine, Isomurrayazoline, Mahanimboline, Mukonicine, Isolongifolene, Murrayakonine A, Murrayakonine B, Murrayakonine C, Murrayakonine D.

Among those listed above, the following are preferred having formula:

| | |
|---|---|
| Mahanine | 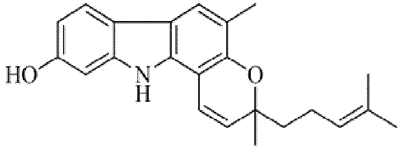 |
| Mahanimbine | 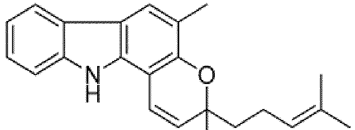 |
| Isomahanine | 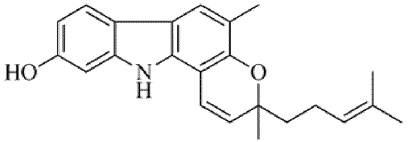 |
| Koenimbine | 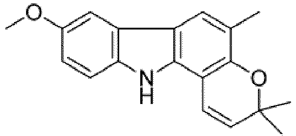 |
| Isolongifolene | 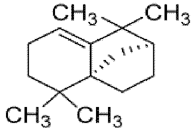 |
| Bismurrayafoline E | 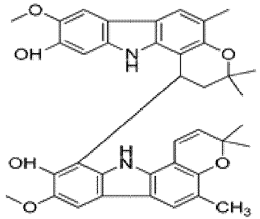 |
| Isomurrayazoline | 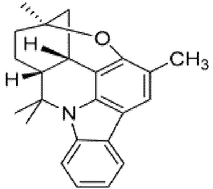 |
| Koenoline | 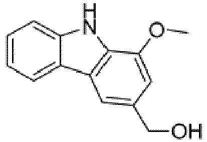 |
| *O*-Methylmurrayamine A | 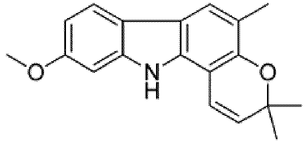 |
| Koenine | 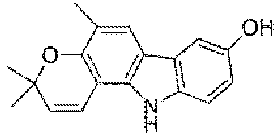 |
| Koenigicine | 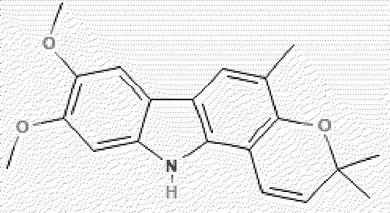 |
| Mukonicine | 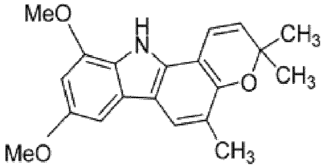 |
| Mahanimbinine | 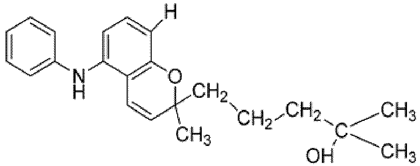 |
| Mahanimboline | 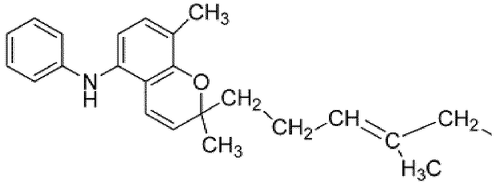 |

In preferred embodiments, carbazole alkaloids of the present invention comprise at least one of Koenigicine, Koenimbine, and Mahanimbine.

In most preferred embodiments, carbazole alkaloids of the present invention comprise a mixture of Koenigicine, Koenimbine, and Mahanimbine.

Carbazole alkaloids have been found in the leaf plant *M. koenigii,* and the roots of *Gylcosmis pentaphylla* and *Clausena heptaphylla.*

Preferably, carbazole alkaloids of the invention are those present in leaves of *Murraya koenigii,* also known as "curry leaves", such as those depicted in the table above.

*M. koenigii* belongs to the *Rutaceae* family, which is commonly used as a medicinally important herb of Indian origin in the Ayurvedic system of medicine. Previous reports have demonstrated that the leaves, roots, and bark of this plant are rich sources of carbazole alkaloids, which produce potent biological activities and pharmacological effects. These include antioxidant, antidiabetic, anti-inflammatory, antitumor, and neuroprotective activities. In recent years, greater attention has been paid to the use of *M*. *koenigii* in traditional medicines and home remedies. On the other hand, limited studies have been conducted for evaluating the pharmacological and medicinal efficacy of *M. koenigii* in promoting health benefits and curing diseases, especially those relating to upper respiratory tract, let alone those ascribable to *S. pyogenes.*

The present invention has surprisingly found and demonstrated that carbazole alkaloids, such as those present in the leaves of *M. koenigii,* show bactericidal effectiveness against *S. pyogenes.*

To this end, the tests carried out have been performed against *S. pyogenes* ATCC 19615. This bacterial strain is isolated from the pharynx of a child following an episode of a sore throat, and is known to be the cause of up to 30% of childhood acute bacterial pharyngitis cases, and up to 20% of asymptomatic children may be carriers at various times of the year (Summer K, et al. Recovery of culturable Streptococcus pyogenes from swabs stored at different temperatures. Environ Microbiol Rep. 2024 Dec; 16(6):e70036; and Minogue TD, et al. Complete Genome Assembly of Streptococcus pyogenes ATCC 19615, a Group A β-Hemolytic Reference Strain. Genome Announc. 2014 Sep 25;2(5):e00976-14), so that the results achieved in said tests *in vitro* against *S. pyogenes* ATCC 19615 are acknowledged to be properly representative of efficacy in the treatment of upper respiratory tract infections caused by *S. pyogenes.*

Preferably, said carbazole alkaloids for the use above described are to be administered in an amount of 5 mg/kg/die to 85 mg/kg/die in case of an adult subject, more preferably 5 mg/kg/die to 20 mg/kg/die, even more preferably 5 mg/kg/die to 10 mg/kg/die.

Preferably, said carbazole alkaloids for the use above described are to be administered in an amount of 1 mg/kg/die to 40 mg/kg/die in case of a paediatric subject, more preferably 1 mg/kg/die to 10 mg/kg/die.

Accordingly, in another aspect, the present invention also concerns a pharmaceutical composition comprising said carbazole alkaloids. These compositions including said natural antimicrobial carbazole alkaloids are found to be desirable alternatives/adjuvants to antibiotics for treating infections, while minimizing antimicrobial resistance. Therefore, this pharmaceutical composition is for use in the treatment of upper respiratory tract infections ascribable to *S. pyogenes.*

The oral route is the most common route of drug administration. Till today, it is still a widely preferred and acceptable route among patients owing to its advantages, such as ease of administration, safeness, non-invasiveness, and convenience for self-administration.

However, one of the obstacles to oral drug delivery experienced by many patients, particularly paediatric and geriatric population, is dysphagia. Dysphagia causes difficulties in swallowing conventional solid oral medicines. Consequently, patients usually try to crush hard tablets or open capsules and mix them with food or water to become swallowing easily. Such behaviour can result in dosing inaccuracy and changing of drug release and absorption, as well as undesirable drug taste palatability.

Therefore, with the aim to overcome these patients' discomfort and improve compliance, carbazole alkaloids of the present invention are administered through a pharmaceutical composition comprising the same. Preferably, the pharmaceutical composition is in the form of a chewing-gum, a chewable tablet, a chewable soft jelly, an oral spray, or a syrup. Chewing gums feature optimal therapeutic time and lower side effects due to their sustained release capability and lower required thresholds for the active compared with other delivery approaches. The convenient use in the oral cavity's local environment and the ability to locally carry multiple actives, in addition to their ability to deliver the proper local dosages of active ingredients, short contact time, biocompatibility, and biodegradable chemical structure, are considered the main advantages of this delivery approach.

Similarly, a chewable tablet is mostly chewed in the mouth, before swallowing. The main aim of the chewable tablet is to give a proper unit dosage form of actives that can easily be given to children or to the elderly who have difficulty swallowing a tablet intact. Chewable tablets are taken to disintegrate smoothly in the mouth at a moderate rate either with or without actual chewing; chewable tablets have a smooth texture upon disintegration, pleasant tasting, and leave no bitter or unpleasant taste. Geriatric and paediatric patients and traveling patients who may not have ready access to water are most in need of easy-swallowing dosage forms like chewable tablets. Moreover, chewing may help the travellers to forget about nausea and the possibility of increasing the absorption because of a high number in the blood vessels in the buccal cavity.

Alternatively, a chewable soft jelly, similar to gelatinous food and confection, can be used to this end. Indeed, also jellies can address swallowing problems, ensure patient safety, and ease of handle and taken without water. Thus, also jellies can improve patient compliance in addition to their flavouring taste and aesthetic pleasant appearance, especially for children. Jellies intended for oral administration mean "non-flowable gelatinous preparations of definite size and shape" and must meet the requirements of dissolution and content uniformity.

The main excipients of jellies are a gelling agent, stabilizer, preservative, and flavouring and sweetening agents. Jellies act as a vehicle for actives which present either as dissolved or dispersed/suspended forms that can release and mix with saliva to be absorbed through gastrointestinal tract mucosa. By choosing the right gelling agent, which can be either natural or synthetic hydrophilic polymers at a suitable concentration, the actives can release immediately or sustained from the jelly vehicle.

Alternatively, the pharmaceutical composition of the invention can be in the form of an oral spray.

Oral sprays offer several benefits, as discussed below.

First, oral sprays are very easy to use and require no measuring or preparation. They can be sprayed directly into the mouth, making them a convenient option for people who are on-the-go or have difficulty swallowing pills.

Also, oral sprays are designed to be absorbed quickly through the mouth's mucous membranes, bypassing the digestive system. This means that they can provide faster and more effective results compared to capsules or tablets, which need to be broken down by the digestive system first.

Additionally, oral sprays typically dispense a consistent dosage with each spray, ensuring that the same quantity of actives is delivered every time.

The pharmaceutical composition of the present invention can also be in the form of a syrup, i.e. one of the most common liquid forms of oral administration, together with solutions, suspensions, emulsions, drops, elixirs, and mouth washes/gargles.

Liquid dosage forms, such as syrups, are prepared by dissolving actives in aqueous and nonaqueous solvents, suspending the actives in appropriate mediums, or by incorporating the actives into oil or water phases. Pharmaceutical liquids therefore come in a variety of formulations.

Liquid dosage forms are available as monophasic and biphasic formulations. While monophasic liquid dosage forms are true (single phase) or colloidal solutions, liquids which consist of two phases are known as biphasic liquids.

For the preparation of liquid dosage forms, such as liquid oral dosage forms, pharmaceutical excipients are added, such as vehicles, stabilizers, preservatives, suspending agents, emulsifying agents, solubilizers, colours, flavours, and mixtures thereof.

Furthermore, liquid dosage forms, such as syrups, offer certain benefits over other specialist dosage forms such as increased patient compliance, faster absorption, and more flexibility in dosing. Some other important advantages of pharmaceutical liquid dosage forms (LDFs) include:
- very useful for those patients who have trouble swallowing complex oral solid dosage forms,
- increase in patient convenience and patient compliance (e.g. liquid oral dosage forms as compared to oral solid dosage forms),
- rate of absorption of a liquid oral dosage form is faster than an oral solid dosage form,
- dosing flexibility, and
- suitability for use by infants, geriatric patients, and those suffering from mental health problems.

In view of the above, depending on the patients' needs, the more suitable pharmaceutical form can be selected for the composition of the invention.

In this regard, the pharmaceutical composition of the invention further comprises pharmaceutically acceptable excipients.

The term "excipient" means a compound or a mixture thereof suitable for use in the formulation of the composition. For example, an excipient for use in a pharmaceutical formulation generally must not cause an adverse response in a user, nor significantly inhibit the effectiveness of the composition.

Suitable excipients are acidifiers, acidity regulators, anti-caking agents, antioxidants, bulking agents, resistance agents, gelling agents, coating agents, modified starches, sequestering agents, thickeners, sweeteners, diluents, disaggregating agents, glidants, colorants, binders, lubricants, stabilisers, adsorbents, preservatives, humectants, flavourings, filmogenic agents, emulsifiers, wetting agents, release retardants, and mixtures thereof.

Preferably, said excipients are potassium sorbate, sodium benzoate, ε-polylysine, sucralose, maltodextrin, citric acid, sodium carbonate, calcium carbonate, magnesium carbonate, magnesium stearate, stearic acid, polyethylene glycol, natural starch, partially hydrolysed starch, modified starch, lactose, calcium phosphate, calcium carbonate, calcium sulphate, polyvinyl pyrrolidone, silica, colloidal silica, precipitated silica, magnesium silicates, aluminium silicates, sodium lauryl sulphate, magnesium lauryl sulphate, methacrylate copolymers, sodium dehydroacetate, xanthan gum, guar gum, tara gum, locust bean gum, fenugreek gum, Arabic gum, alginic acid, sodium alginate, propylene glycol alginate, sodium croscarmellose, polyvinylpolypyrrolidone, glyceryl behenate, titanium dioxide, indigo carmine, cellulose, modified cellulose, calcium carboxymethylcellulose, sodium carboxymethylcellulose, microcrystalline cellulose, ethyl cellulose, gelatine, hydroxyethyl cellulose, hydroxypropyl cellulose, polydextrose, carrageenan, methylcellulose, sucrose, sucrose esters, sorbitol, xylitol, dextrose, fructose, maltitol, tragacanth gum, pectin, agar-agar, carboxypolymethylene, hydroxypropyl methyl cellulose, mannitol, silicon dioxide, or mixtures thereof. Preferred excipients include an acidifier, such as citric acid, a sweetener, such as sucralose, and an anti-caking agent, such as silicon dioxide.

The pharmaceutical composition of the present invention can also comprise additional ingredients, such as vitamins, plant extracts, glycosaminoglycans, lactic acid bacteria, probiotics, prebiotics, postbiotics, essential oils, spices, herbs, minerals, peptides, lipids and derivatives, amino acids and amino acid derivatives, proteins, GAG, sugars and derivatives, enzymes, phytosterol and sterols, neurotransmitters and derivatives, nucleic acids and derivatives, plant pigments and derivatives, organic sulphur compounds, lactones and derivatives, and mixtures thereof.

The pharmaceutical composition of the present invention can also include any one of the herbal extracts and preparations listed in Annex 1 of the Ministerial Decree of 10 August 2018, concerning the regulation of the use of herbal substances and preparations in food supplements, that describe the following properties: 1. has an emollient and soothing action on the oropharyngeal mucosa and on the tone of the voice; 2. contributes to the fluidity of bronchial secretions; 3. contributes to the wellbeing of the nose and throat; 4. well-being and tropism of the mucous membranes; 5. promotes the functionality of the first respiratory tract.

The pharmaceutical composition of the present invention can be prepared by methods known in the art.

In the pharmaceutical composition, carbazole alkaloids are present as such or in the form of ground leaves of *M. koenigii,* alcoholic extracts of *M. koenigii,* powdered extracts of *M. koenigii,* liquid extracts of *M. koenigii,* glyceric extracts of *M. koenigii,* oleolites and essential oils of *M. koenigii,* microencapsulated dry extracts of *M. koenigii,* liposomes and nano-liposomes of *M. koenigii,* phytosomes of *M. koenigii,* solid-liquid micro and nanoparticles of *M. koenigii,* micronized dry extracts of *M. koenigii,* hydrosols and spray-dried extracts of *M. koenigii,* infusions of *M. koenigii,* decoctions of *M. koenigii,* soft extracts of *M. koenigii,* dry extracts in oleoresin of *M. koenigii,* suspensions of *M. koenigii,* or combinations thereof.

It is understood that also all the possible combinations of the preferred aspects of the carbazole alkaloids, pharmaceutical composition and uses thereof are described above and are therefore likewise preferred.

It should furthermore be understood that all the aspects specified as preferred and advantageous for the carbazole alkaloids should therefore likewise be considered preferred and advantageous for the pharmaceutical composition and uses thereof.

Below are working examples of the present invention provided for illustrative, nonlimiting purposes.

### EXAMPLES

### Example 1

### HPLC/DAD analysis of carbazole alkaloids

Dried and ground leaves of *M. koenigii* were subjected to extraction with methanol, and the resulting extract was analysed by liquid chromatography.

The quantitative analysis was performed by comparing the areas obtained with the calibration lines of three individual standards. The following active ingredients were identified:

| Koenigicine (ppm) | Koenimbine (ppm) | Mahanimbine (ppm) |
|---|---|---|
| 942 | 450 | 1150 |

### Example 2

### Investigation of antibacterial activity of M. koenigii leaves against S. pyogenes by agar well diffusion method

Dried and ground leaves of *M. koenigii* were subjected to extraction with DMSO, and the resulting extract was studied as follows.

The extraction studies revealed that DMSO was the solvent that provided no antibacterial effect (and thus properly used as negative control on *S. pyogenes*) and the highest extraction of bioactive carbazole alkaloids.

The agar well diffusion method was used for the assessment of the antibacterial activity of *M. koenigii* leaves against *S. pyogenes* (ATCC 19615). Six DMSO extracts of *M. koenigii* leaves from different geographical origins were tested.

Antibacterial assay was performed on M17 agar (Liofilchem SRL, Roseto degli Abruzzi, Italy). An overnight anaerobic culture of *S. pyogenes* grown at 37°C, was diluted in order to obtain a suspension containing approximately 1×10⁸ CFU/ml. Each agar plate was spread with the microbial suspension. Using a sterile agar gel puncher, equidistant wells were created and filled with 0.1 ml of each sample. The plates were allowed to stand for 30 minutes to allow pre-diffusion of the tested samples, and thereafter incubated at 37°C for 24 h under anaerobic conditions. After incubation, the diameter of any zone of inhibition surrounding each well was measured. Each experiment was performed in triplicates and the means were calculated.

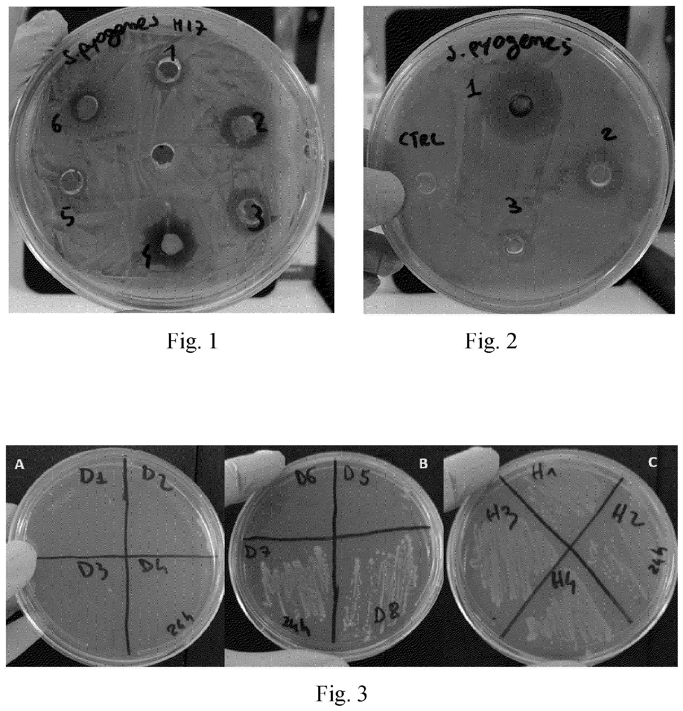

Agar well diffusion test showed that three out of six *M. koenigii* leaves extracts screened had antibacterial effect against *S. pyogenes* (Figure 1). The DMSO extract with the highest yield in carbazole alkaloids was tested at three different concentrations (100 mg/ml, 20 mg/ml, 5 mg/ml) on *S. pyogenes.* The maximum zone of inhibition (20 mm) was observed at a concentration of 100 mg/ml (Figure 2). A zone of inhibition of 20 mm or more, is categorized as very strong by several studies.

The results of the antibacterial activity are reported in Table 1. The extract exhibited an appreciably high inhibitory effect against *S. pyogenes:*

**Table 1: Antibacterial activity of DMSO extract of M. koenigii leaves against S. pyogenes.**

| **Sample** | **Inhibition zone (mm)** |
|---|---|
| Extract 100 mg/ml | 20 |
| Extract 20 mg/ml | 14 |
| Extract 5 mg/ml | 8 |
| DMSO | - |

### Example 3

### TTC assay for MIC and MBC determination of M. koenigii leaves against S. pyogenes

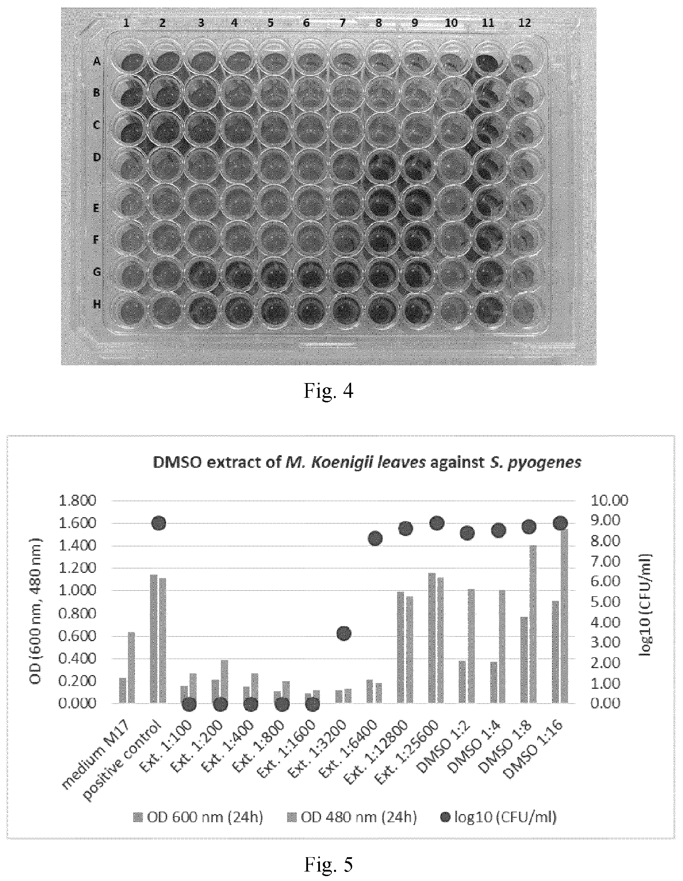

Minimum Inhibitory Concentration (MIC) and Minimum Bactericidal Concentration (MBC) were determined in a 96 well microtiter plate (Brand plates Brand GMBH, Germany Pure grade TM-S) using the broth microdilution method and 2,3,5-Triphenyl Tetrazolium Chloride (TTC) (Sigma-Aldrich, St. Louis, MO) as colorimetric indicator. In the presence of living bacteria, the colourless TTC is reduced to red formazan. The absorbance of formazan, measured at 480 nm, is directly proportional to the amount of living bacteria. Two different stock solutions of *M. koenigii* leaves were prepared in DMSO at concentrations of 100 mg/ml (1:10) and 20 mg/ml (1:50). Two-fold dilution method was performed in order to obtain a concentration range of 0.04 mg/ml to 10 mg/ml for the 20 mg/ml stock solution and of 0.2 mg/ml to 50 mg/ml for the 100 mg/ml stock solution. An overnight anaerobic culture of *S. pyogenes* grown at 37°C was adjusted to a final concentration of 1x10⁷ CFU/ml in M17 broth (Liofilchem SRL, Roseto degli Abruzzi, Italy) and added to the microplate wells containing different concentrations of the extract, in order to obtain a final inoculum of approximately 1x10⁶ CFU/well. The microplate was incubated at 37°C under anaerobic conditions for 24 h. After incubation, the microplate was observed for change in colour and the absorbance at 600 nm and 480 nm were measured by a microplate reader (TECAN Infinite M200 Pro, Switzerland). To discriminate between bactericidal/bacteriostatic activity of the extract, 100 µl aliquots from each tested dilution were used to calculate the number of viable cells (CFU/ml) by plate count method and 10 µl aliquots from the same wells were streaked on M17 agar plates (Figure 3). TTC assay revealed that the extract had a strong inhibitory activity against *S. pyogenes,* with a MIC value of 0.31 mg/ml (dilution 1:3200), while causing a percentage of inhibition of approximately 60%. A complete inhibition of growth was observed up to dilution 1/1600 equivalent to MBC value of 0.63 mg/ml. No inhibitory effect on bacterial growth was observed for the solvent DMSO, confirming the results of agar well diffusion test. TTC assay is depicted in Figure 4, where a picture of the microplate used for the TTC assay is reported. OD values (600 nm, 480 nm), log10 (CFU/ml) values and percentage inhibition are shown in Table 2, Figure 5.

**Table 2: Antibacterial activity of DMSO extract of M. koenigii leaves against S. pyogenes. All results are the mean of triplicates wells. OD 480 nm (absorbance of TTC), OD 600 nm (absorbance of bacterial cells). Medium M17 (blank), positive control (S. pyogenes without M. koenigii), Ext. (Extract).**

| **Microplate well** | **OD 600 nm (24 h)** | **OD 480 nm (24 h)** | **log10 (CFU/ml)** | **% Inhibition** |
|---|---|---|---|---|
| medium M17 | 0.232 | 0.631 | 0.00 | - |
| positive control | 1.142 | 1.111 | 8.91 | - |
| Ext. 1:100 | 0.158 | 0.265 | 0.00 | 100.00 |
| Ext. 1:200 | 0.213 | 0.386 | 0.00 | 100.00 |
| Ext. 1:400 | 0.151 | 0.272 | 0.00 | 100.00 |
| Ext. 1:800 | 0.112 | 0.198 | 0.00 | 100.00 |
| Ext.1:1600 | 0.096 | 0.120 | 0.00 | 100.00 |
| Ext. 1:3200 | 0.121 | 0.130 | 3.48 | 60.98 |
| Ext. 1:6400 | 0.214 | 0.178 | 8.18 | 8.25 |
| Ext. 1:12800 | 0.995 | 0.951 | 8.65 | 2.89 |
| Ext. 1:25600 | 1.159 | 1.116 | 8.89 | 0.25 |
| DMSO 1:2 | 0.381 | 1.012 | 8.44 | 5.29 |
| DMSO 1:4 | 0.375 | 1.006 | 8.54 | 4.12 |
| DMSO 1:8 | 0.771 | 1.402 | 8.72 | 2.14 |
| DMSO 1:16 | 0.916 | 1.547 | 8.89 | 0.25 |

### Example 4

### Comparison between antibacterial activity of M. koenigii leaves and amoxicillin

A stock solution of amoxicillin (Sigma-Aldrich, St. Louis, USA) was prepared at a concentration of 0.25 mg/ml in DMSO.

The antibacterial properties against *S. pyogenes* of the plant extract alone, amoxicillin alone and their combination were evaluated by TTC assay as mentioned above. For the experiment, the 20 mg/ml stock solution of *M. koenigii* leaves was used and a solution containing 0.25 mg/ml amoxicillin and 20 mg/ml extract was prepared. The results confirmed a MIC value of 0.31 mg/ml at dilution 1:3200 and a MBC value of 0.63 mg/ml at dilution 1:1600.

Amoxicillin showed a MIC value of 0.016 mg/ml (dilution 1:16) and determined a complete inhibition of growth of *S. pyogenes* up to dilution 1/8 equivalent to MBC value of 0.031 mg/ml.

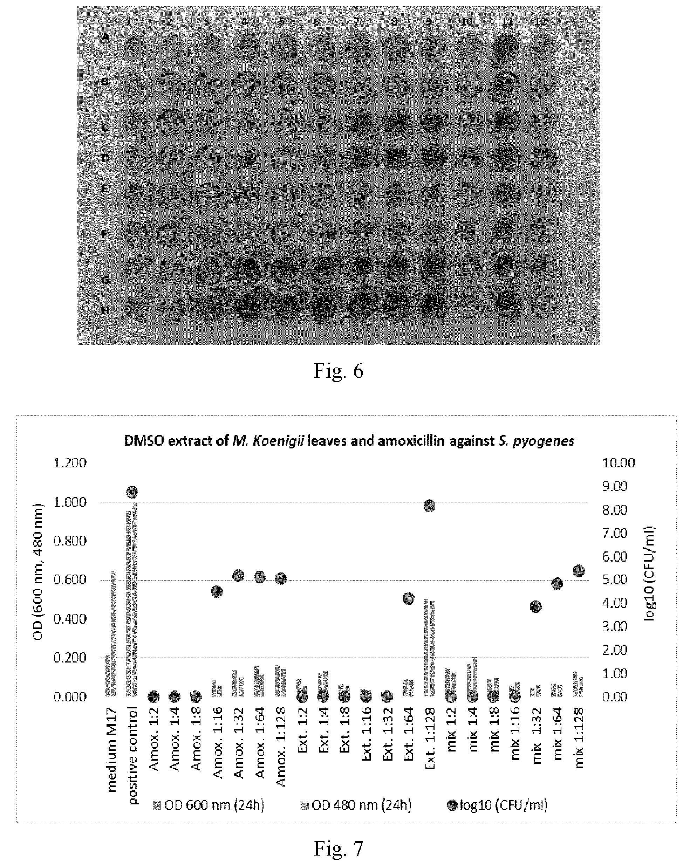

The combination of *M. koenigii* extract and amoxicillin exhibited a higher bactericidal effect (100% growth inhibition up to dilution 1:16) compared to amoxicillin alone (48.57%). Moreover, the combination showed a higher percentage of inhibition (55.76%) compared to amoxicillin alone (40.61%) at dilution 1:32. Similar effects were observed at subsequent dilutions. No inhibitory effect was reported for the solvent DMSO, confirming previous results. TTC assay is depicted in Figure 6. OD values (600 nm, 480 nm), log10 (CFU/ml) values and percentage of inhibition are shown in Table 3, Figure 7.

**Table 3: Comparison of antibacterial activity of DMSO extract of M. koenigii leaves and amoxicillin against S. pyogenes. All results are the mean of duplicates wells. OD 480 nm (absorbance of TTC), OD 600 nm (absorbance of bacterial cells). Medium M17 (blank), positive control (S. pyogenes without M. koenigii), Ext. (Extract), Amox. (Amoxicillin), Mix (Extract and Amoxicillin).**

| **Microplate well** | **OD 600 nm (24 h)** | **OD 480 nm (24 h)** | **log10 (CFU/ml)** | **% Inhibition** |
|---|---|---|---|---|
| medium M17 | 0.210 | 0.647 | 0.00 | - |
| positive control | 0.957 | 0.998 | 8.76 | - |
| Amox. 1:2 | 0.002 | 0.003 | 0.00 | 100.00 |
| Amox. 1:4 | 0.011 | 0.001 | 0.00 | 100.00 |
| Amox. 1:8 | 0.026 | 0.005 | 0.00 | 100.00 |
| Amox. 1:16 | 0.088 | 0.057 | 4.51 | 48.57 |
| Amox. 1:32 | 0.137 | 0.098 | 5.20 | 40.61 |
| Amox. 1:64 | 0.158 | 0.120 | 5.12 | 41.49 |
| Amox. 1:128 | 0.161 | 0.140 | 5.08 | 42.02 |
| Ext. 1:100 | 0.092 | 0.054 | 0.00 | 100.00 |
| Ext. 1:200 | 0.122 | 0.133 | 0.00 | 100.00 |
| Ext. 1:400 | 0.065 | 0.051 | 0.00 | 100.00 |
| Ext. 1:800 | 0.039 | 0.036 | 0.00 | 100.00 |
| Ext. 1:1600 | 0.025 | 0.025 | 0.00 | 100.00 |
| Ext. 1:3200 | 0.090 | 0.086 | 3.62 | 58.64 |
| Ext. 1:6400 | 0.501 | 0.492 | 8.18 | 6.66 |
| Mix 1:2 | 0.145 | 0.126 | 0.00 | 100.00 |
| Mix 1:4 | 0.167 | 0.202 | 0.00 | 100.00 |
| Mix 1:8 | 0.091 | 0.096 | 0.00 | 100.00 |
| Mix 1:16 | 0.057 | 0.070 | 0.00 | 100.00 |
| Mix 1:32 | 0.046 | 0.058 | 3.88 | 55.76 |
| Mix 1:64 | 0.069 | 0.060 | 4.85 | 44.69 |
| Mix 1:128 | 0.130 | 0.102 | 5.39 | 38.48 |
| DMSO 1:2 | 0.099 | 0.104 | 8.13 | 7.18 |
| DMSO 1:4 | 0.229 | 0.182 | 8.23 | 6.04 |
| DMSO 1:8 | 0.444 | 0.417 | 8.29 | 5.36 |
| DMSO 1:16 | 0.799 | 0.832 | 8.53 | 2.60 |

The results obtained from this study provided evidence that the DMSO extract of *M. koenigii* leaves exhibited a strong antibacterial activity against *S. pyogenes* with a zone of inhibition diameter of 20 mm and a MIC value of 0.31 mg/ml. It should be appreciated that strong microbial inhibitors show MIC values equal or lower than 0.50 mg/ml. Moreover, the extract was able to completely prevent bacterial growth up to a concentration of 0.63 mg/ml (MBC). It should be appreciated that an active is considered bactericidal when the ratio MBC/MIC is equal or lower than 4.

Furthermore, the antimicrobial activity of the combination of amoxicillin and the extract was evaluated. The addition of *M. koenigii* leaves to amoxicillin increased the antibacterial properties of amoxicillin alone against *S. pyogenes.* These findings could have significant implications for the nutraceutical and pharmaceutical industries. The use of plant extracts, either alone or combined with antibiotics, could help in dealing with the present crisis of antibiotic resistance.

Furthermore, our study highlighted the importance of several factors, such as the starting raw material, the choice of solvent, and solvent material/ratio, to obtain the highest yield of bioactive carbazole alkaloids from *M. koenigii* leaves.

Overall, these data support the use of *M. koenigii* leaves as natural antibacterial agent against *S. pyogenes,* thus accordingly being useful as active agents in the treatment of infections caused by *S. pyogenes,* such as tonsillitis and pharyngitis, even in paediatric age, owing to the safety of natural herbal source, simultaneously preventing, or at least minimizing, the onset of AMR in this bacterial pathogen.

### Example 5

### Chewing-gum formulation for adults

A chewing-gum comprising dried and ground leaves of *M. koenigii,* Vitamin C and plant extracts of Meadowsweet and Mallow was prepared, wherein:

| | For each chewing-gum | NVR% |
|---|---|---|
| *M. koenigii* | 50 mg | ------ |
| Vitamin C | 50 mg | 62.5% |
| Meadowsweet | 50 mg | ------ |
| Mallow | 5 mg | ------ |
| Excipients | Balance to 1.7 g | |

HOW TO USE: It is recommended to chew each chewing-gum at least 10-15 minutes to secure the release of the active agents.

PHYSIOLOGICAL EFFECTS: Mallow has an emollient and soothing action on the oropharyngeal mucosa and on the tone of the voice and contributes to the fluidity of bronchial secretions.

NUTRITIONAL EFFECTS: Vitamin C contributes to the maintenance of the normal function of the immune system.

### Example 6

### Chewing-gum formulation for children

A chewing-gum for children comprising dried and ground leaves of *M. koenigii,* Vitamin C and plant extracts of Blackcurrant and Mallow was prepared, wherein:

| | For each chewing-gum | NVR% |
|---|---|---|
| *M. koenigii* | 30 mg | ------ |
| Vitamin C | 50 mg | 62.5% |
| Blackcurrant | 50 mg | ------ |
| Mallow | 5 mg | ------ |
| Excipients | Balance to 1.7 g | |

HOW TO USE: It is suggested to chew the coated chewable tablets as needed without exceeding the recommended daily dose.

PHYSIOLOGICAL EFFECTS: Chamomile contributes to the functionality of the mucous membranes of the respiratory system; Mallow has an emollient and smoothing action on the oropharyngeal mucosa and on the tone of the voice and contributes to the fluidity of bronchial secretions.

NUTRITIONAL EFFECTS: Vitamin C contributes to the maintenance of the normal function of the immune system.

### Example 7

### Chewable tablet formulation for children

A chewable tablet for children, comprising dried and ground leaves of *M. koenigii,* Chamomile and Mallow plant extracts, Hyaluronic Acid and Vitamin C was prepared, wherein:

| | For each tablet | NVR% |
|---|---|---|
| *M. koenigii* | 10 mg | ------ |
| Hyaluronic acid | 5 mg | ------ |
| Vitamin C | 35 mg | 43.75% |
| Chamomile | 10 mg | ------ |
| Mallow | 5 mg | ------ |
| Excipients | Balance to 1 g | |

HOW TO USE: It is suggested to chew the coated chewable tablets as needed without exceeding the recommended daily dose.

PHYSIOLOGICAL EFFECTS: Chamomile contributes to the functionality of the mucous membranes of the respiratory system; Mallow has an emollient and soothing action on the oropharyngeal mucosa and on the tone of the voice and contributes to the fluidity of bronchial secretions.

NUTRITIONAL EFFECTS: Vitamin C contributes to the maintenance of the normal function of the immune system.

### Example 8

### Chewable tablet formulation for adults

A chewable tablet for adults, comprising dried and ground leaves of *M. koenigii,* Chamomile and Mallow plant extracts, Hyaluronic Acid and Vitamin C was prepared, wherein:

| | For each tablet | NVR% |
|---|---|---|
| *M. koenigii* | 50 mg | ------ |
| Hyaluronic acid | 5 mg | ------ |
| Vitamin C | 120 mg | 150% |
| Chamomile | 20 mg | ------ |
| Mallow | 10 mg | ------- |
| Excipients | Balance to 1.2 g | |

HOW TO USE: It is suggested to chew the chewable tablets as needed without exceeding the recommended daily dose.

PHYSIOLOGICAL EFFECTS: Chamomile contributes to the functionality of the mucous membranes of the respiratory system; Mallow has an emollient and soothing action on the oropharyngeal mucosa and on the tone of the voice and contributes to the fluidity of bronchial secretions.

NUTRITIONAL EFFECTS: Vitamin C contributes to the maintenance of the normal function of the immune system.

### Example 9

### Oral spray formulation for adults

An oral spray for adults, comprising extract of *M. koenigii,* Honey and plant extracts of Meadowsweet and Blackcurrant was prepared wherein:

| | For bottle |
|---|---|
| *M. koenigii* | 60 mg |
| Honey | 600 mg |
| Meadowsweet | 10 mg |
| Blackcurrant | 150 mg |
| Excipients | Balance to 20 mL |

PHYSIOLOGICAL EFFECTS: Blackcurrant contributes to the wellbeing of nose and throat.

### Example 10

### Oral spray formulation for children

An oral spray for children, extract of *M. koenigii,* Honey and plant extracts of Chamomile and Blackcurrant was prepared wherein:

| | For bottle |
|---|---|
| *M. koenigii* | 30 mg |
| Honey | 600 mg |
| Chamomile | 30 mg |
| Blackcurrant | 150 mg |
| Excipients | Balance to 20 mL |

PHYSIOLOGICAL EFFECTS: Chamomile contributes to the functionality of the mucous membranes of the respiratory system. Blackcurrant contributes to the wellbeing of the nose and throat.

### Example 11

### Syrup formulation for children and adults

A syrup for children and adults, extract of *M. koenigii,* Vitamin C and plant extracts of Chamomile, Blackcurrant and Drosera was prepared wherein:

| | For bottle |
|---|---|
| *M. koenigii* | 60 mg |
| Vitamin C | 35 mg |
| Chamomile | 30 mg |
| Blackcurrant | 150 mg |
| Drosera | 200 mg |
| Excipients | Balance to 100 mL |

PHYSIOLOGICAL EFFECTS: Chamomile contributes to the functionality of the mucous membranes of the respiratory system. Blackcurrant contributes to the wellbeing of the nose and throat. Drosera contributes to the fluidity of bronchial secretions, has an emollient and soothing action on the oropharyngeal mucosa and on the tone of the voice.

NUTRITIONAL EFFECTS: Vitamin C contributes to the maintenance of the normal function of the immune system.

### Example 12

### Qualitative and quantitative analyses of M. koenigii leaves by HPLC/DAD

The qualitative and quantitative analyses of *M. koenigii* were performed by liquid chromatography with DAD detector. A mix containing each single standard at known concentrations was prepared for quantitative analysis. The standards of Koenigicine, Koenimbine and Mahanimbine were purchased from Sigma-Aldrich (St. Louis, USA). Water (H₂O) was obtained from a water producer (FullTech Instruments, Rome, Italy), whereas Methanol (MeOH), Acetonitrile (ACN), Acetic Acid Glacial (AcA), Ethanol (EtOH), Dimethyl Sulfoxide (DMSO), Chloroform (CHCl3) were purchased by Levanchimica (Bari, Italy).

The chromatographic separation was performed by an HPLC/DAD system series 1200 from Agilent (Agilent Technologies, Santa Chiara, California, USA); the analytical column was a Kinetex C18 5um, 100A (150 mm x 3.00 mm i.d.) from Phenomenex (Phenomenex SRL, Bologna; Italy) equipped with a security guard column. The mobile phase consisted of H₂O containing 0.1% of AcA and ACN. The elution of the analytes was carried out with a gradient method: initial condition was 40% of ACN, then brought to 90% in 10 minutes and kept for 8 minutes. Koenigicine and Koenimbine were quantified at 295 nm, Mahanimbine was quantified at 285 nm.

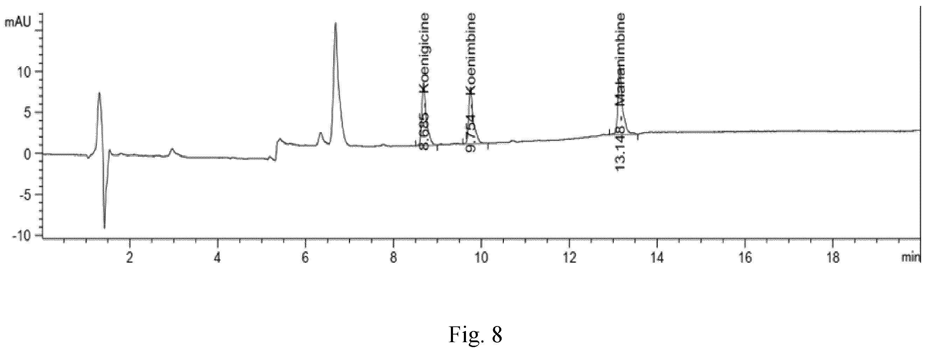

Koenigicine and Koenimbine standard were weighed separately and solubilized in MeOH, while Mahanimbine was weighed and solubilized in CHCl₃, in order to have a concentration of 0.5 mg/ml for each standard solution (500 ppm). An aliquot of each solution was withdrawn in order to prepare a mix standard solution and a calibration curve from 0.5 ppm to 4.0 ppm in MeOH was created for the quantification of samples. The separation of standards was obtained in 16 minutes, as shown in Figure 8.

Samples of dried and ground leaves of *M. koenigii* were weighed, solubilized in MeOH and sonicated for 30 minutes. An aliquot was withdrawn and dissolved in MeOH.

The analysis of release of single components from the chewing gum of Example 5 was important to determine the activity and the efficacy of dried and ground leaves of *M. koenigii* within said chewing gum, once in a physiological saliva environment. For this analysis three chewers (i.e. healthy adult volunteers enrolled for testing the inventive chewing gum) were asked to chew 4 chewing gums of Example 5, for 5, 10, 15 and 20 minutes, respectively. Each chewed bolus of the resulting 4 chewing tests, was frozen overnight and then weighed, ground and added with MeOH. The supernatant was filtered and analysed by HPLC/DAD. In Table 4 below, the release results are reported, being focused on the main actives of *M. koenigii.*

**Table 4: Percentage of Koenigicine, Koenimbine, Mahanimbine vs. different chewing time points.**

| All results are the average of the values obtained from the three chewers. | | | | |
|---|---|---|---|---|
| **Chewing time points (min)** | **% Koenigicine release** | **% Koenimbine release** | **% Mahanimbine release** | **% Total Alkaloids release** |
| 0.0 | 0.0 | 0.0 | 0.0 | 0.0 |
| 5.0 | 52.7 | 51.9 | 54.7 | 53.8 |
| 10.0 | 49.5 | 48.5 | 51.5 | 50.6 |
| 15.0 | 57.2 | 56.3 | 59.7 | 58.6 |
| 20.0 | 52.4 | 51.5 | 54.7 | 53.7 |

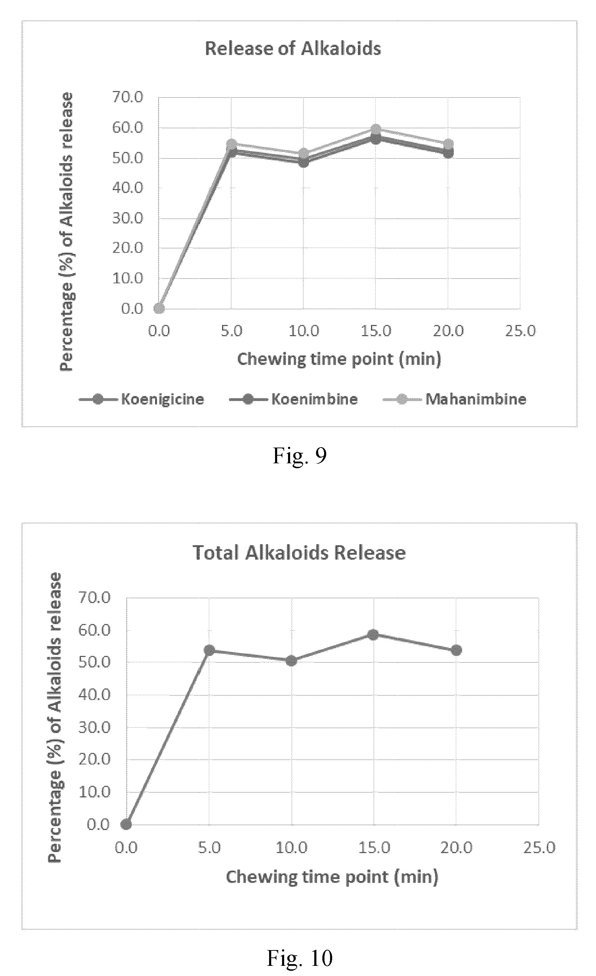

The results are also depicted in Figure 9, wherein the percentage of Koenigicine, Koenimbine, Mahanimbine is reported for the different chewing time points. The single points are the average of the values obtained from the three chewers.

In Figure 10, the percentage of total alkaloids release is reported at the different chewing time points. The single points are the average of the values obtained from the three chewers.

It should be appreciated that the relevant actives are properly and significantly released *in vivo,* i.e. in a physiological saliva environment, thus confirming that the pharmaceutical composition of the invention allow to exploit the beneficial effects of the carbazole alkaloids for treating upper respiratory tract infections ascribable to *S. pyogenes.*

### Example 13

### Evaluation of antibacterial activity of Koenimbine, Koenigicine and Mahanimbine standards against S. pyogenes by agar well diffusion method

The agar well diffusion method was used for the assessment of the antibacterial activity of Koenimbine, Koenigicine and Mahanimbine standards against *S. pyogenes* (ATCC 19615). The standards of Koenigicine, Koenimbine and Mahanimbine were purchased from Sigma-Aldrich (St. Louis, USA). Each standard was weighed separately, solubilized in methanol and diluted in DMSO. The final concentrations tested were 3 µg/ml for Koenigicine, 4 µg/ml for Koenimbine and 6 µg/ml for Mahanimbine.

Antibacterial assay was performed on M17 agar (Liofilchem SRL, Roseto degli Abruzzi, 25 Italy). An overnight anaerobic culture of *S. pyogenes,* grown at 37°C, was diluted approximately to obtain 1×10⁸ CFU/ml and spread on M17 agar. Using a sterile agar gel puncher, equidistant wells were created and filled with 0.1 ml of each extraction solvent. The plate was allowed to stand for 30 minutes to allow pre-diffusion of the tested samples, and thereafter incubated at 37°C. The zone of inhibition around each well was measured in mm.

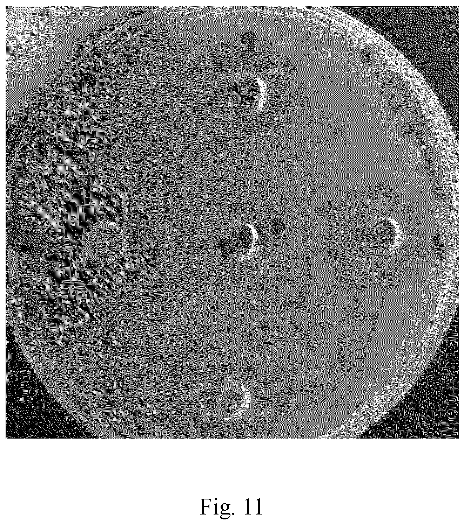

As shown in Figure 11, each standard effectively inhibited *S. pyogenes* growth with an inhibition zone of about 18 mm for Koenimbine and 20 mm for Koenigicine and Mahanimbine. The well in the centre was filled with DMSO as a negative control.

## Claims

1. Carbazole alkaloids for use as active agents in the treatment of upper respiratory tract infections ascribable to *Streptococcus pyogenes.*

2. The carbazole alkaloids for use of claim 1, wherein carbazole alkaloids comprise at least one of Mahanine, Mahanimbine, Isomahanine, Koenimbine, Isolongifolene, Bismurrayafoline E, Isomurrayazoline, Koenoline, O-Methylmurrayamine A, Koenine, Koenigicine, Mukonicine, Mahanimbinine, and Mahanimboline.

3. The carbazole alkaloids for use of claim 2, wherein carbazole alkaloids comprise at least one of Koenigicine, Koenimbine, and Mahanimbine.

4. The carbazole alkaloids for use of any one of claims 1-3, wherein carbazole alkaloids comprise a mixture of Koenigicine, Koenimbine, and Mahanimbine.

5. The carbazole alkaloids for use of any one of claims 1-4, wherein said carbazole alkaloids of the invention are those present in leaves of *Murraya koenigii.*

6. The carbazole alkaloids for use of any one of claims 1-5, wherein said carbazole alkaloids are to be administered in an amount of 5 mg/kg/die to 85 mg/kg/die in case of an adult subject, more preferably 5 mg/kg/die to 20 mg/kg/die, even more preferably 5 mg/kg/die to 10 mg/kg/die.

7. The carbazole alkaloids for use of any one of claims 1-5, wherein said carbazole alkaloids are to be administered in an amount of 1 mg/kg/die to 40 mg/kg/die in case of a paediatric subject, more preferably 1 mg/kg/die to 10 mg/kg/die.

8. A pharmaceutical composition for use in the treatment of upper respiratory tract infections ascribable to *Streptococcus pyogenes,* the composition comprising the carbazole alkaloids of any one of claims 1-7, and pharmaceutically acceptable excipients.

9. The pharmaceutical composition for use of claim 8, being in the form of a chewing-gum, a chewable tablet, a chewable soft jelly, an oral spray, or a syrup.

10. The pharmaceutical composition for use of claim 8 or 9, wherein carbazole alkaloids are present as such or in the form of ground leaves of *M. koenigii,* alcoholic extracts of *M. koenigii,* powdered extracts of *M. koenigii,* liquid extracts of *M. koenigii,* glyceric extracts of *M. koenigii,* oleolites and essential oils of *M. koenigii,* microencapsulated dry extracts of *M. koenigii,* liposomes and nano-liposomes of *M. koenigii,* phytosomes of *M. koenigii,* solid-liquid micro and nanoparticles of *M. koenigii,* micronized dry extracts of *M. koenigii,* hydrosols and spray-dried extracts of *M. koenigii,* infusions of *M. koenigii,* decoctions of *M. koenigii,* soft extracts of *M. koenigii,* dry extracts in oleoresin of *M. koenigii,* suspensions of *M. koenigii,* or combinations thereof.
